# FASCICULE DE BREVET EUROPEEN

(11) **EP 3 256 100 B1**
(45) Date de publication et mention de la délivrance du brevet: **29.06.2022**
(21) Numéro de dépôt: 15810699.7
(22) Date de dépôt: 07.12.2015
(51) Int. Cl.: A61K 8/44, A61K 8/46, A61Q 5/02, A61Q 19/10

(54) **COMPOSITION COSMETIQUE COMPRENANT DES OLEFINE SULFONATES LINEAIRES ET DES TENSIOACTIFS ANIONIQUES NON OXYALKYLENES**
KOSMETISCHE ZUBEREITUNG ENTHALTEND LINEARE OLEFIN SULFONATE UND NICHT OXYALKYLIERTE ANIONISCHE TENSIDE
COSMETIC COMPOSITION COMPRISING LINEAR SULFONATE OLEFINS AND NON OXYALKYLATED ANIONIC SURFACTANTS

(30) Priorité: 12.12.2014 FR 1462322
(43) Date de publication de la demande: 20.12.2017
(73) Titulaire: L'Oréal, 75008 Paris (FR)
(72) Inventeur: DARRAS, Marie-Florence, 93400 Saint Ouen (FR); DUSSAULT, Lydia, 93400 SaintOuen (FR); MATHONNEAU, Estelle, 93400 Saint Ouen (FR); PLOS, Grégory, 93400 Saint Ouen (FR)
(74) Mandataire: Casalonga
(86) Numéro de dépôt international: PCT/FR2015/053344
(87) Numéro de publication internationale: WO 2016/092189

(56) Documents cités:
- WO-A2-2011/007174
- DATABASE GNPD [Online] MINTEL; 1 septembre 2014 (2014-09-01), Vogue International: "Shampoo", XP002742928, Database accession no. 2665843
- DATABASE GNPD [Online] MINTEL; 1 juillet 2014 (2014-07-01), "Dry or Sensitised Skin Bar Soap", XP002742929, Database accession no. 2550117
- DATABASE GNPD [Online] MINTEL; 2 juin 2014 (2014-06-02), "Liquid Face & Body Cleanser", XP002742930, Database accession no. 2487863
- DATABASE GNPD MINTEL; 1 September 2014 (2014-09-01), Vogue International: "Shampoo",
- DATABASE GNPD [Online] MINTEL; 2 July 2012 (2012-07-02), "Volumizing Shampoo", Database accession no. 1828427
- DATABASE GNPD [Online] MINTEL; 1 October 2012 (2012-10-01), "Professional Shampoo", Database accession no. 1901592
- DATABASE GNPD [Online] MINTEL; 1 June 2010 (2010-06-01), "Shampoo", Database accession no. 1336633

## Description

La présente invention concerne une composition cosmétique comprenant une ou plusieurs alpha-oléfine sulfonates linéaires, en association avec un ou plusieurs tensioactifs anioniques additionnels particuliers et avec un ou plusieurs tensioactifs additionnels choisis parmi les tensioactifs amphotères et les tensioactifs non ioniques; l'invention concerne également un procédé de traitement cosmétique mettant en oeuvre ladite composition.

Les chevelures ont tendance à perdre certaines de leurs qualités sous l'action de facteurs tels que le regraissage naturel, la sueur, l'élimination de squames, la pollution ou l'humidité notamment. L'aspect visuel ainsi que le toucher des cheveux peut ainsi être dégradé. Le regraissage, par exemple, alourdit les cheveux qui ont alors tendance à se mettre en paquets. Les cheveux peuvent être plus difficiles à coiffer, et avoir une brillance grasse ou un toucher ciré désagréable. Il en est de même pour la peau qui a tendance à perdre certaines de ses qualités sous l'action de facteurs tels que la sueur, l'élimination de squames, la pollution, le climat, le chauffage. L'aspect visuel peut être dégradé et ainsi conduire à une peau terne, desséchée, et un sentiment d'inconfort peut apparaitre.

Pour le lavage des matières kératiniques telles que la peau et les cheveux, l'utilisation de compositions cosmétiques détergentes de type shampooing ou gel-douche, à base essentiellement d'agents tensioactifs, est courante. Ces compositions sont généralement appliquées sur les matières kératiniques de préférence mouillées, et la mousse générée par massage ou friction avec les mains ou un gant de toilette permet, après rinçage à l'eau, l'élimination des diverses salissures initialement présentes sur les cheveux ou la peau.

Ces compositions contiennent des teneurs substantielles en tensioactifs détergents qui, pour permettre de formuler des compositions cosmétiques ayant un bon pouvoir lavant et aptes à éliminer les salissures (sébum, sueur, pollution, etc.), les squames ou les pellicules, doivent notamment conférer à celles-ci un bon pouvoir moussant. Les tensioactifs utiles à cet effet sont généralement de type anionique, non ionique ou amphotère, et particulièrement de type anionique.

Les compositions cosmétiques de lavage des matières kératiniques les plus courantes contiennent souvent des tensioactifs anioniques de type sulfate, qui sont de très bons tensioactifs détergents, mais peuvent se révéler peu respectueux des matières kératiniques à laver, en particulier en ayant un effet desséchant desdites matières, voire en impactant négativement le conditionnement des cheveux ou de la peau. En effet, ces tensioactifs peuvent altérer, au fur et à mesure des applications, les propriétés cosmétiques des cheveux ou de la peau, ce qui conduit à la nécessité d'utiliser également des agents conditionneurs comme des polymères cationiques, des silicones ou des huiles non siliconées. Les tensioactifs sulfates peuvent également engendrer chez certains consommateurs sensibles, des problèmes de tolérance, notamment au niveau de la peau et/ou des yeux (picotements, tiraillement).

Il existe donc un besoin de proposer des compositions cosmétiques, notamment de type shampoing ou gel-douche, ayant un bon pouvoir détergent et notamment un bon pouvoir moussant, tout en étant le plus possible exemptes de tensioactifs sulfates.

Toutefois, les compositions cosmétiques lavantes exemptes de tensioactifs sulfates doivent généralement comprendre des quantités importantes d'autres agents tensioactifs, afin d'obtenir l'abondance et la qualité de mousse recherchées. Or, l'emploi de grandes quantités de tensioactifs est peu souhaitable, car il augmente le coût des compositions et peut également conduire à des problèmes de tolérance chez les consommateurs les plus sensibles.

Ainsi, il existe donc un réel besoin de disposer de compositions cosmétiques qui présentent des propriétés de mousse satisfaisantes, sans avoir à employer de tensioactifs sulfates ni de quantités importantes de tensioactifs non sulfates.

Ces compositions doivent également posséder de bonnes propriétés détergentes ou lavantes, présenter une bonne tolérance notamment vis-à-vis de la peau, des muqueuses, du cuir chevelu et des yeux, tout en conduisant à un bon conditionnement des matières kératiniques.

Enfin, pour éviter les coulures à l'application et notamment les coulures dans les yeux, les compositions détergentes de type shampooings ou gel-douches doivent de préférence généralement présenter une texture épaissie, mais sans que leur épaississement ne génère des problèmes de stabilité de la composition.

La Demanderesse a maintenant découvert qu'une composition cosmétique contenant une association particulière de tensioactifs permettait d'atteindre les objectifs exposés ci-dessus.

La présente invention a donc pour objet une composition cosmétique comprenant :
- (i) une ou plusieurs alpha-oléfine sulfonates linéaires,
- (ii) un ou plusieurs tensioactifs anioniques non oxyalkylénés, différents des composés (i), présents dans la composition en une quantité allant de 1 à 20% en poids par rapport au poids total de la composition; la composition comprenant au moins un tensioactif anionique non oxyalkyléné, différent des composés (i), choisis parmi les acylsarcosinates, les alkylsulfosuccinates en C6-C24, et les (C6-C24)acyliséthionates et
- (iii) au moins un tensioactif non ionique choisi parmi les (alkyl C6-C24)polyglycosides, les esters d'acides gras en C8-C30 du sorbitane éthoxylés, les alcools gras en C8-C30 polyéthoxylés, les esters d'acides gras en C8-C30 polyoxyéthylénés, les alcanolamides d'acides gras en C8-C30, et leurs mélanges, et au moins un tensioactif amphotère choisi parmi les alkyl(C8-C20)bétaïnes, les alkyl(C8-C20)amidoalkyl(C1-C6)bétaïnes, les alkyl(C8-C20) amphoacétates et les alkyl(C8-C20) amphodiacétates, et leurs mélanges ; la quantité totale des tensioactifs additionnels non ioniques et amphotères allant de 1,5 à 12% en poids, par rapport au poids total de la composition ;
ladite composition ne comprenant pas de tensioactif anionique comprenant de groupement sulfate.

Les compositions selon l'invention permettent l'obtention d'une mousse abondante et de très bonne qualité, notamment crémeuse, et ceci de manière rapide. Notamment, elles fournissent une mousse homogène, qui présente une bonne tenue dans le temps. La mousse formée à partir de la composition selon l'invention se répartit facilement et de manière uniforme sur les matières kératiniques.

La présente invention permet d'obtenir des performances de moussage optimales malgré l'absence de tensioactifs non sulfates dans la composition. Ainsi, la présente invention permet de formuler des compositions qui, à teneur en tensioactifs équivalentes aux compositions de l'art antérieur, présentent des performances de moussage supérieures. Surtout, la présente invention permet de formuler des compositions qui peuvent contenir des quantités de tensioactifs moins importantes que les compositions de l'art antérieur, tout en présentant des performances de moussage au moins équivalentes, voire supérieures.

En outre, la composition selon l'invention possède de bonnes propriétés cosmétiques, et procure notamment un bon conditionnement des matières kératiniques et notamment des cheveux, y compris lorsque que celles-ci sont sensibilisées.

La composition selon l'invention peut également présenter une texture épaissie, qui permet sa répartition sur les cheveux en évitant les coulures dans les yeux, par exemple; on a également constaté que la composition selon l'invention était particulièrement stable, bien que de texture épaissie.

En mettant en oeuvre l'invention, il est ainsi possible d'obtenir des compositions moussantes et épaissies sans avoir à ajouter d'agents épaississants autres que du sel (NaCl par exemple), qui pourraient nuire à la stabilité de ladite composition et/ou à ses propriétés moussantes.

De préférence, la composition selon l'invention présente une viscosité mesurée à 25°C, 1 atm. comprise entre 1 et 20 Pa.s, mieux entre 3 et 15 Pa.s, et tout particulièrement entre 4 et 12 Pa.s.

La viscosité est de préférence mesurée à l'aide d'un appareil rhéomètre Haake Mars en géométrie cône-plan diamètre 60 mm / 1° (titane). La température est régulée par un plan à effet Peltier à 25°C, le taux de cisaillement est de 200 s⁻¹.

La viscosité peut également être mesurée à l'aide d'un Rhéomat 180, à 25°C, 1 atm., avec un mobile 3 ou 4, la vitesse de rotation étant de 200 tr/min et le temps de mesure de 10 minutes, le taux de cisaillement étant de 200 s⁻¹.

Dans la présente description, l'expression "au moins un" est équivalente à l'expression "un ou plusieurs" et peut y être substituée.

Dans la présente description, l'expression "compris entre" est équivalente à l'expression "allant de" et peut y être substituée; dans ces expressions, les bornes sont considérées comme incluses.

### (i) α-oléfine sulfonates linéaires (ou alpha-oléfine sulfonates linéaires)

La composition cosmétique selon l'invention comprend donc une ou plusieurs alpha-oléfine sulfonates linéaires, de préférence comprenant 8 à 28 atomes de carbone, notamment de 10 à 24 atomes de carbone, encore mieux de 12 à 20 atomes de carbone, en particulier de 14 à 18 atomes de carbone.

Lesdites alpha-oléfine sulfonates sont des composés connus et sont notamment décrits dans l'Encyclopédie des Industries Chimiques Ullmann's ou encore dans le brevet US8211850. Ces composés sont généralement obtenus par sulfonation d'alpha-oléfines à chaine longue.

Les α-oléfine sulfonates linéaires selon l'invention comprennent généralement, de manière connue, un mélange d'alcènes sulfonates linéaires, notamment de formule (A), optionnellement en mélange avec des hydroxyalcanes sulfonates linéaires, notamment de formule (B).

Lesdits alcènes sulfonates linéaires peuvent donc être de formule (A) :

R-CH₂-CH=CH-(CH₂)n-SO₃M

dans laquelle :
- R est un radical alkyle linéaire saturé comprenant de 4 à 30 atomes de carbone, notamment de 6 à 20 atomes de carbone, voire de 8 à 18 atomes de carbone, encore mieux de 10 à 14 atomes de carbone ;
- n est un entier compris entre 0 et 10, de préférence entre 1 et 4, et encore mieux n=1 ;
- M étant un cation cosmétiquement acceptable, notamment choisi parmi le cation ammonium, les cations issus des métaux alcalins ou alcalinoterreux, les cations issus d'amines organiques comme les alcanolamines; de préférence ceux issus des métaux alcalins ; et notamment Na+ ou K+.

De préférence, R représente un radical alkyle linéaire comprenant 8 à 14 atomes de carbone, notamment de 10 à 12 atomes de carbone.

De préférence, M est issu d'un métal alcalin, notamment Na+ ou K+.

De préférence, les alcènes sulfonates linéaires selon l'invention sont de formule : 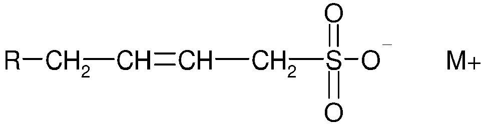 dans laquelle :
- R est un radical alkyle linéaire saturé comprenant de 4 à 20 atomes de carbone, notamment de 6 à 18 atomes de carbone, voire de 8 à 14 atomes de carbone, encore mieux de 10 à 12 atomes de carbone ;
- M étant un cation cosmétiquement acceptable, notamment choisi parmi le cation ammonium, les cations issus des métaux alcalins ou alcalinoterreux, les cations issus d'amines organiques comme les alcanolamines; de préférence ceux issus des métaux alcalins et notamment Na+ ou K+.

Les hydroxyalcanes sulfonates linéaires peuvent être de formule (B) :

(B) R'-CH₂-CH(OH)-CH₂-(CH₂)n'-SO₃M'

dans laquelle :
- R' est un radical alkyle linéaire saturé comprenant de 4 à 30 atomes de carbone, notamment de 6 à 20 atomes de carbone, voire de 8 à 18 atomes de carbone, encore mieux de 10 à 14 atomes de carbone ;
- n' est un entier compris entre 0 et 10, de préférence entre 1 et 4, et encore mieux n'=1 ;
- M' étant un cation cosmétiquement acceptable, notamment choisi parmi le cation ammonium, les cations issus des métaux alcalins ou alcalinoterreux, les cations issus d'amines organiques comme les alcanolamines; de préférence ceux issus des métaux alcalins, et notamment Na+ ou K+.

De préférence, R' représente un radical alkyle linéaire comprenant 8 à 14 atomes de carbone, notamment de 10 à 12 atomes de carbone.

De préférence, M' est issu d'un métal alcalin, notamment Na+ ou K+.

De préférence, les hydroxyalcanes sulfonates linéaires sont de formule : 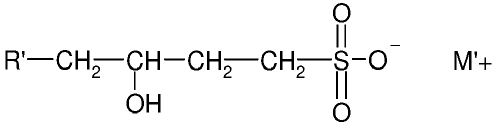 dans laquelle :
- R' est un radical alkyle linéaire saturé comprenant de 4 à 20 atomes de carbone, notamment de 6 à 18 atomes de carbone, voire de 8 à 14 atomes de carbone, encore mieux de 10 à 12 atomes de carbone ;
- M' étant un cation cosmétiquement acceptable, notamment choisi parmi le cation ammonium, les cations issus des métaux alcalins ou alcalinoterreux, les cations issus d'amines organiques comme les alcanolamines; de préférence ceux issus des métaux alcalins et notamment Na+ ou K+.

De préférence, R et R' sont identiques.

De préférence, M et M' sont identiques.

De préférence, les α-oléfine sulfonates linéaires selon l'invention sont choisies parmi les α-oléfine sulfonates linéaires comprenant 8 à 28 atomes de carbone, notamment de 10 à 24 atomes de carbone, encore mieux de 12 à 20 atomes de carbone, en particulier de 14 à 18 atomes de carbone ; en particulier de métaux alcalins, notamment de sodium.

On peut notamment citer comme produits commerciaux, ceux proposés sous la dénomination BIO-TERGE AS-40A ou BIO-TERGE AS-40HA par la société STE-PAN, ou CALSOFT AOS-40 par la société PILOT CHEMICAL, ou encore NANSA LSS38/AV par la société HUNTSMAN.

De préférence, la composition cosmétique selon l'invention comprend lesdites alpha-oléfine sulfonates linéaires en une quantité allant de 1 à 20% en poids, notamment de 4 à 18% en poids, de préférence de 6 à 16% en poids, par rapport au poids total de la composition cosmétique.

### (ii) Tensioactifs anioniques non oxyalkylénés additionnels

La composition selon l'invention comprend également un ou plusieurs tensioactifs anioniques non oxyalkylénés, différents des alpha-oléfines sulfonates linéaires ci-dessus ; la composition comprenant au moins un tensioactif anionique non oxyalkyléné, différent des alpha-oléfines sulfonates linéaires ci-dessus choisi parmi les acylsarcosinates, les alkylsulfosuccinates en C6-C24, et les (C6-C24)acyliséthionates, ainsi que les sels de ces composés et leurs mélanges.

On entend par tensioactif anionique, un tensioactif ne comportant à titre de groupements ioniques ou ionisables que des groupements anioniques

Dans la présente description, on qualifie une entité comme étant "anionique" lorsqu'elle possède au moins une charge négative permanente ou lorsqu'elle peut être ionisée en une entité chargée négativement, dans les conditions d'utilisation de la composition de l'invention (milieu, pH par exemple) et ne comprenant pas de charge cationique.

On entend par tensioactif non oxyalkyléné, un tensioactif ne comprenant pas de motif oxyde d'alkylène, en particulier de motif oxyde d'éthylène (OCH₂CH₂), de motif oxyde de propylène ou de motif oxyde de butylène.

Lorsque le tensioactif anionique est sous forme de sel, ledit sel peut être choisi parmi les sels de métaux alcalins tels que le sel de sodium ou de potassium, les sels d'ammonium, les sels d'amines et en particulier d'aminoalcools, et les sels de métaux alcalino-terreux tel que le sel de magnésium. Comme exemple de sels d'aminoalcools, on peut citer les sels de mono-, di- et triéthanolamine, les sels de mono-, di- ou tri-isopropanolamine, les sels de 2-amino 2-méthyl 1-propanol, 2-amino 2-méthyl 1,3-propanediol et tris(hydroxyméthyl)amino méthane. On utilise de préférence les sels de métaux alcalins ou alcalinoterreux, et en particulier les sels de sodium ou de magnésium.

Lesdits tensioactifs anioniques sont choisis parmi, seuls ou en mélange :
- les acylsarcosinates notamment en C6-C24, voire en C12-C20, tels que les palmitoylsarcosinates et les lauroylsarcosinates, et en particulier le palmitoylsarcosinate de sodium ou le lauroylsarcosinate de sodium ;
- les alkylsulfosuccinates en C6-C24, notamment en C12-C20, notamment les laurylsulfosuccinates, en particulier de sodium;
- les (C6-C24)acyliséthionates, de préférence les (C12-C18) acyliséthionates, et tout particulièrement les cocoyliséthionates, notamment de sodium ;
en particulier sous forme de sels de métaux alcalins ou alcalino-terreux, d'ammonium, ou d'aminoalcool.

Le ou les tensioactifs anioniques non oxyalkylénés différents des composés (i) sont présents dans la composition en une quantité allant de 1 à 20% en poids, notamment de 1,5 à 15% en poids, mieux de 2 à 10% en poids, encore mieux de 3 à 10% en poids, par rapport au poids total de la composition.

Le rapport pondéral de la quantité d'a-oléfine sulfonates linéaires (i) sur la quantité de tensioactifs anioniques non oxyalkylénés (ii) différents des composés (i) est avantageusement supérieur ou égal à 1 (rapport pondéral (i)/(ii) ≥ 1), de préférence compris entre 1,2 et 12, notamment entre 1,5 et 10, voire entre 1,8 et 8, encore mieux allant de 2 à 7, voire de 2 à 4.

La composition selon l'invention est dite 'sulfate-free', c'est-à-dire qu'elle ne comprend pas (0%) de tensioactif anionique comprenant de groupement sulfate, tel que par exemple les alkylsulfates et les alkyléthersulfates.

Dans un mode de réalisation particulier, la composition peut comprendre, à titre de tensioactif optionnel, un ou plusieurs tensioactifs anioniques oxyalkylénés, qui peuvent être choisis parmi :
- les acides alkyléthercarboxyliques, les acides alkyl(aryl en C6-30)éthercarboxyliques, les acides alkylamidoéthercarboxyliques, les alkyléthersul-fosuccinates, les alkylamidoéthersulfosuccinates, ainsi que les sels de ces composés; ces composés étant polyoxyalkylénés, notamment polyoxyéthylénés et comportant alors de préférence de 1 à 50 motifs oxyde d'éthylène, mieux de 2 à 10 motifs oxyde d'éthylène ; les groupes alkyle et/ou acyle de ces composés comportant de 6 à 30 atomes de carbone, notamment de 12 à 28, encore mieux de 14 à 24, voire de 16 à 22, atomes de carbone; le groupe aryle désignant de préférence un groupe phényle ou benzyle;
- les alkylsulfonates, les alkylamidesulfonates, les alkylarylsulfonates, les paraffine-sulfonates, les alkylsulfosuccinates, les alkylsulfoacétates, les N-acyltaurates, les acyliséthionates, les alkylsulfolaurates ; ainsi que les sels de ces composés; ces composés étant polyoxyalkylénés, notamment polyoxyéthylénés et comportant alors de préférence de 1 à 50 motifs oxyde d'éthylène, mieux de 2 à 10 motifs oxyde d'éthylène ; les groupes alkyle de ces composés comportant de 6 à 30 atomes de carbone, notamment de 12 à 28, encore mieux de 14 à 24, voire de 16 à 22, atomes de carbone; le groupe aryle désignant de préférence un groupe phényle ou benzyle.

Dans un mode de réalisation particulier de l'invention, la composition ne comprend pas (0%) de tensioactif anionique oxyéthyléné.

### (iii) Tensioactifs non ioniques et amphotères additionnels

La composition selon l'invention comprend également au moins un tensioactif non ionique et au moins un tensioactif amphotère.

Le tensioactif non ionique est choisi parmi les (alkyl C6-C24)polyglycosides, et plus particulièrement les (alkyl C8-C18)polyglycosides, les esters d'acides gras en C8-C30 du sorbitane éthoxylés, les alcools gras en C8-C30 polyéthoxylés, les esters d'acides gras en C8-C30 polyoxyéthylénés, ces composés ayant de préférence 2 à 150 moles d'oxyde d'éthylène; les alcanolamides d'acides gras en C8-C30 tels que les monoalcanolamides d'acides gras en C8-C30, en particulier les monoéthanolamides ou monoisopropanolamides d'acide gras en C8-C30, et leurs mélanges; et encore mieux le tensioactif non ionique est choisi parmi les alcanolamides d'acides gras en C8-C30, tels que les monoalcanolamides d'acides gras en C8-C30, en particulier les monoéthanolamides ou monoisopropanolamides d'acide gras en C8-C30.

Les tensioactifs amphotères sont choisis parmi les alkyl(C8-C20)bétaïnes, les alkyl(C8-C20)amidoalkyl(C1-C6)bétaïnes, les alkyl(C8-C20) amphoacétates et les alkyl(C8-C20) amphodiacétates, et leurs mélanges.

La composition selon l'invention comprend le ou lesdits tensioactifs additionnels non ioniques et amphotères, en une quantité totale allant de 1,5 à 12% en poids, voire de 2 à 10% en poids, par rapport au poids total de la composition.

La composition selon l'invention comprend un ou plusieurs tensioactifs amphotères, qui sont de préférence présents en une quantité allant de 0,5 à 15% en poids, notamment allant de 1 à 10% en poids, préférentiellement allant de 1,5 à 8% en poids, par rapport au poids total de la composition.

Dans un mode de réalisation particulier, la composition selon l'invention comprend une teneur totale en tensioactifs (oléfines sulfonates linéaires (i) + anioniques (ii) + amphotères et non ioniques (iii) + cationiques le cas échant) inférieure ou égale à 25% en poids, en particulier inférieure ou égale à 22% en poids, notamment inférieure ou égale à 20% en poids, encore mieux inférieure ou égale à 18% en poids, par rapport au poids total de ladite composition.

### Electrolyte

La composition selon l'invention peut comprendre en outre une ou plusieurs électrolytes, par exemple choisies parmi les sels minéraux des métaux alcalins ou alcalinoterreux tels que le chlorure de sodium, le sulfate de sodium, le chlorure de magnésium, le sulfate de magnésium, et encore plus préférentiellement choisies parmi les sels minéraux des métaux monovalents alcalins ou alcalinoterreux, et en particulier le chlorure de sodium.

La composition selon l'invention comprend de préférence ladite ou lesdites électrolytes en une quantité allant de 0,01 à 10% en poids, notamment allant de 0,05 à 5% en poids, mieux allant de 0,1 à 3% en poids, voire allant de 0,15 à 2% en poids, par rapport au poids total de la composition.

### Polymères amphotères ou cationiques

La composition selon l'invention peut comprendre en outre un ou plusieurs polymères choisis parmi les polymères amphotères ou cationiques, ainsi que leurs mélanges.

On entend par "polymère cationique", tout polymère comprenant des groupements cationiques et/ou des groupements ionisables en groupements cationiques. De préférence, le polymère cationique est hydrophile ou amphiphile. Les polymères cationiques préférés sont choisis parmi ceux qui contiennent des motifs comportant des groupements amines primaires, secondaires, tertiaires et/ou quaternaires pouvant soit faire partie de la chaîne principale polymère, soit être portés par un substituant latéral directement relié à celle-ci.

Les polymères cationiques susceptibles d'être utilisés ont de préférence une masse molaire moyenne en poids (Mw) comprise entre 500 et 5.10⁶ environ, de préférence comprise entre 10³ et 3.10⁶ environ.

Parmi les polymères cationiques, on peut citer plus particulièrement :
(1) les homopolymères ou copolymères dérivés d'esters ou d'amides acryliques ou méthacryliques et comportant au moins un des motifs de formule suivante : 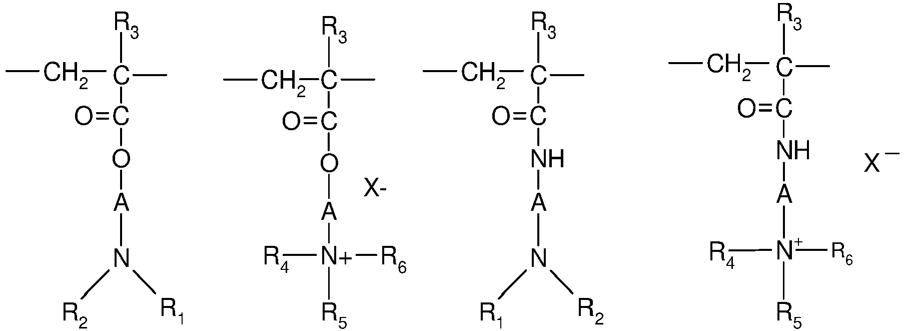 dans lesquelles:
   - R3, identiques ou différents, désignent un atome d'hydrogène ou un radical CH3;
   - A, identiques ou différents, représentent un groupe divalent alkyle, linéaire ou ramifié, de 1 à 6 atomes de carbone, de préférence 2 ou 3 atomes de carbone ou un groupe hydroxyalkyle de 1 à 4 atomes de carbone ;
   - R4, R5, R6, identiques ou différents, représentent un groupe alkyle ayant de 1 à 18 atomes de carbone ou un radical benzyle; de préférence un groupe alkyle ayant de 1 à 6 atomes de carbone;
   - R1 et R2, identiques ou différents, représentent un atome d'hydrogène ou un groupe alkyle ayant de 1 à 6 atomes de carbone, de préférence méthyle ou éthyle;
   - X désigne un anion dérivé d'un acide minéral ou organique tel qu'un anion méthosulfate ou un halogénure tel que chlorure ou bromure.

Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.

Parmi ces copolymères de la famille (1), on peut citer :
- les copolymères d'acrylamide et de diméthylaminoéthyl méthacrylate quaternisé au sulfate de diméthyle ou avec un halogénure de diméthyle, tels que celui vendu sous la dénomination HERCOFLOC par la société HERCULES,
- les copolymères d'acrylamide et de chlorure de méthacryloyloxyéthyltriméthyl-ammonium, tels que ceux vendus sous la dénomination BINA QUAT P 100 par la société CIBA GEIGY,
- le copolymère d'acrylamide et de méthosulfate de méthacryloyloxyéthyltriméthyl-ammonium, tel que celui vendu sous la dénomination RETEN par la société HERCULES,
- les copolymères vinylpyrrolidone/acrylate ou méthacrylate de dialkylaminoalkyle, quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthylaminoéthyle/ vinylcaprolactame/ vinylpyrrolidone, tel que le produit vendu sous la dénomination GAFFIX VC 713 par la société ISP,
- les copolymères vinylpyrrolidone/ méthacrylamidopropyldimethylamine, tels que ceux commercialisés sous la dénomination STYLEZE CC 10 par ISP;
- les copolymères vinylpyrrolidone/ méthacrylamide de diméthylaminopropyle quaternisé, tel que le produit vendu sous la dénomination "GAFQUAT HS 100" par la société ISP,
- les polymères, de préférence réticulés, de sels de méthacryloyloxyalkyl(C1-C4) trialkyl(C1-C4)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo- ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion comprenant 50% en poids dudit copolymère dans de l'huile minérale. Cette dispersion est commercialisée sous le nom de "SALCARE^{®} SC 92" par la société CIBA. On peut également utiliser un homopolymère réticulé du chlorure de méthacryloyloxyéthyl triméthylammonium comprenant environ 50% en poids de l'homopolymère dans de l'huile minérale ou dans un ester liquide. Ces dispersions sont commercialisées sous les noms "SALCARE^{®} SC 95" et "SALCARE^{®} SC 96" par la société CIBA.

(2) Les polysaccharides cationiques, notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.

Les dérivés d'éthers de cellulose comportant des groupements ammonium quaternaires sont notamment décrits dans FR1492597, et on peut citer les polymères commercialisés sous la dénomination "UCARE POLYMER JR" (JR 400 LT, JR 125, JR 30M) ou "LR" (LR 400, LR 30M) par la Société AMERCHOL. Ces polymères sont également définis dans le dictionnaire CTFA comme des ammonium quaternaires d'hydroxyéthylcellulose ayant réagi avec un époxyde substitué par un groupement triméthylammonium.

Les copolymères de cellulose cationiques ou les dérivés de cellulose greffés avec un monomère hydrosoluble d'ammonium quaternaire, sont décrits notamment dans le brevet US4131576, et on peut citer les hydroxyalkylcelluloses, comme les hydroxyméthyl-, hydroxyéthyl- ou hydroxypropyl celluloses greffées notamment avec un sel de méthacryloyléthyl triméthylammonium, de méthacrylamidopropyl triméthylammonium, de diméthyl-diallylammonium. Les produits commercialisés répondant à cette définition sont plus particulièrement les produits vendus sous la dénomination "Celquat L 200" et "Celquat H 100" par la Société National Starch. Les gommes de galactomannane cationiques sont décrites plus particulièrement dans les brevets US3589578 et US4031307, et on peut citer les gommes de guar comprenant des groupements cationiques trialkylammonium. On utilise par exemple des gommes de guar modifiées par un sel (par exemple un chlorure) de 2,3-époxypropyl triméthylammonium. De tels produits sont commercialisés notamment sous les dénominations JAGUAR C13 S, JAGUAR C 15, JAGUAR C 17 ou JAGUAR C162 par la société RHODIA.

(3) les polymères constitués de motifs pipérazinyle et de radicaux divalents alkylène ou hydroxyalkylène à chaînes linéaires ou ramifiées, éventuellement interrompues par des atomes d'oxygène, de soufre, d'azote ou par des cycles aromatiques ou hétérocycliques, ainsi que les produits d'oxydation et/ou de quaternisa-tion de ces polymères.

(4) les polyaminoamides solubles dans l'eau, préparés en particulier par polycondensation d'un composé acide avec une polyamine; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé; l'agent réticulant étant utilisé dans des proportions allant de 0,025 à 0,35 mole par groupement amine du polyaminoamide; ces polyaminoamides peuvent être alcoylés ou s'ils comportent une ou plusieurs fonctions amines tertiaires, quaternisés.

(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone; le rapport molaire entre le polyalkylène polyamine et l'acide dicarboxylique étant de préférence compris entre 0,8:1 et 1,4:1; le polyaminoamide en résultant étant amené à réagir avec l'épichlorhydrine dans un rapport molaire d'épichlorhydrine par rapport au groupement amine secondaire du polyaminoamide compris de préférence entre 0,5:1 et 1,8:1. Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.

(7) les cyclopolymères d'alkyl diallyl amine ou de dialkyl diallyl ammonium tels que les homopolymères ou copolymères comportant comme constituant principal de la chaîne des motifs répondant aux formules (I) ou (II) : 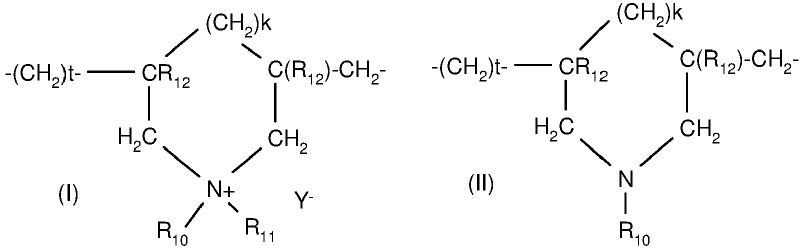 dans lesquelles
- k et t sont égaux à 0 ou 1, la somme k + t étant égale à 1 ;
- R12 désigne un atome d'hydrogène ou un radical méthyle ;
- R10 et R11, indépendamment l'un de l'autre, désignent un groupement alkyle ayant de 1 à 6 atomes de carbone, un groupement hydroxyalkyle dans lequel le groupement alkyle a 1 à 5 atomes de carbone, un groupement amidoalkyle en C1-C4; ou bien R10 et R11 peuvent désigner conjointement avec l'atome d'azote auquel ils sont rattachés, des groupement hétérocycliques, tels que pipéridinyle ou morpholinyle ; R10 et R11, indépendamment l'un de l'autre, désignent de préférence un groupement alkyle ayant de 1 à 4 atomes de carbone.
- Y- est un anion tel que bromure, chlorure, acétate, borate, citrate, tartrate, bisulfate, bisulfite, sulfate, phosphate.

On peut citer plus particulièrement l'homopolymère de sels (par exemple chlorure) de diméthyldiallylammonium par exemple vendu sous la dénomination "MERQUAT 100" par la société NALCO (et leurs homologues de faibles masses molaires moyenne en poids) et les copolymères de sels (par exemple chlorure) de diallyldiméthylammonium et d'acrylamide commercialisés notamment sous la dénomination "MERQUAT 550" ou "MERQUAT 7SPR".

(8) les polymères de diammonium quaternaire comprenant des motifs récurrents de formule : 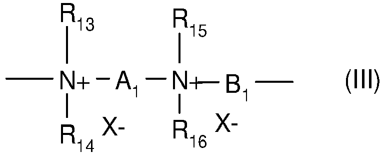 dans laquelle :
- R13, R14, R15 et R16, identiques ou différents, représentent des radicaux aliphatiques, alicycliques, ou arylaliphatiques comprenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles comprenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
- A1 et B1 représentent des groupements divalents polyméthyléniques comprenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
- X- désigne un anion dérivé d'un acide minéral ou organique;
   Etant entendu que A1, R13 et R15 peuvent former avec les deux atomes d'azote auxquels ils sont rattachés un cycle pipérazinique ;
   en outre si A1 désigne un radical alkylène ou hydroxyalkylène linéaire ou ramifié, saturé ou insaturé, B1 peut également désigner un groupement (CH2)n-CO-D-OC-(CH2)n- dans lequel D désigne :
      a) un reste de glycol de formule -O-Z-O-, où Z désigne un radical hydrocarboné linéaire ou ramifié ou un groupement répondant à l'une des formules suivantes: -(CH2-CH2-O)x-CH2-CH2- et -[CH2-CH(CH3)-O]y-CH2-CH(CH3)- où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical divalent -CH2-CH2-S-S-CH2-CH2- ;
      d) un groupement uréylène de formule : -NH-CO-NH- ;
   De préférence, X- est un anion tel que le chlorure ou le bromure. Ces polymères ont une masse molaire moyenne en nombre (Mn) généralement comprise entre 1000 et 100000.

On peut citer plus particulièrement les polymères qui sont constitués de motifs récurrents répondant à la formule : 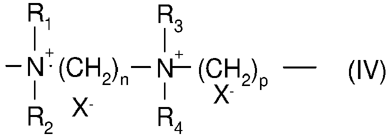 dans laquelle R1, R2, R3 et R4, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.

Un composé de formule (IV) particulièrement préféré est celui pour lequel R1, R2, R3 et R4 représentent un radical méthyle, n=3, p=6 et X = CI, dénommé Hexadi-methrine chloride selon la nomenclature INCI (CTFA).

(9) les polymères de polyammonium quaternaires comprenant des motifs de formule (V): 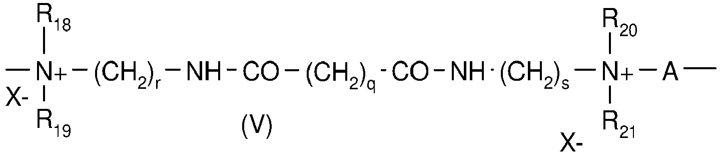 dans laquelle :
- R18, R19, R20 et R21, identiques ou différents, représentent un atome d'hydrogène ou un radical méthyle, éthyle, propyle, β-hydroxyéthyle, β-hydroxypropyle ou -CH2CH2(OCH2CH2)pOH, où p est égal à 0 ou à un nombre entier compris entre 1 et 6, sous réserve que R18, R19, R20 et R21 ne représentent pas simultanément un atome d'hydrogène,
- r et s, identiques ou différents, sont des nombres entiers compris entre 1 et 6,
- q est égal à 0 ou à un nombre entier compris entre 1 et 34,
- X- désigne un anion tel qu'un halogénure,
- A désigne un radical d'un dihalogénure ou représente de préférence -CH2-CH2-O-CH2-CH2-.

On peut par exemple citer les produits "Mirapol^{®} A 15", "Mirapol^{®} AD1", "Mirapol^{®} AZ1" et "Mirapol^{®} 175" vendus par la société Miranol.

(10) Les polymères quaternaires de vinylpyrrolidone et de vinylimidazole tels que par exemple les produits commercialisés sous les dénominations Luviquat^{®} FC 905, FC 550 et FC 370 par la société B.A.S.F.

(11) Les polyamines comme le Polyquart^{®} H vendu par COGNIS, référencé sous le nom de "POLYETHYLENEGLYCOL (15) TALLOW POLYAMINE" dans le dictionnaire CTFA.

(12) les polymères comportant dans leur structure :
(a) un ou plusieurs motifs répondant à la formule (A) suivante : 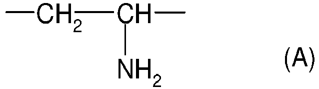
(b) éventuellement un ou plusieurs motifs répondant à la formule (B) suivante :

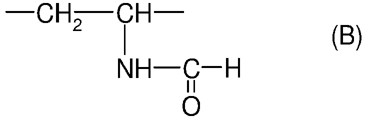

Autrement dit, ces polymères peuvent être notamment choisis parmi les homo- ou copolymères comportant un ou plusieurs motifs issus de la vinylamine et éventuellement un ou plusieurs motifs issus du vinylformamide.

De préférence, ces polymères cationiques sont choisis parmi les polymères comportant, dans leur structure, de 5 à 100% en moles de motifs répondant à la formule (A) et de 0 à 95% en moles de motifs répondant à la formule (B), préférentiellement de 10 à 100% en moles de motifs répondant à la formule (A) et de 0 à 90% en moles de motifs répondant à la formule (B).

Ces polymères peuvent être obtenus par exemple par hydrolyse partielle du polyvinylformamide. Cette hydrolyse peut se faire en milieu acide ou basique.

La masse moléculaire moyenne en poids dudit polymère, mesurée par diffraction de la lumière, peut varier de 1000 à 3.000.000 g/mole, de préférence de 10 000 à 1.000.000 et plus particulièrement de 100 000 à 500.000 g/mole.

La densité de charge cationique de ces polymères peut varier de 2 meq/g à 20 meq/g, de préférence de 2,5 à 15 et plus particulièrement de 3,5 à 10 meq/g.

Les polymères comportant des motifs de formule (A) et éventuellement des motifs de formule (B) sont notamment vendus sous la dénomination LUPAMIN par la société BASF, tels que par exemple, et de manière non limitative, les produits proposés sous la dénomination LUPAMIN 9095, LUPAMIN 5095, LUPAMIN 1095, LUPAMIN 9030 (ou LUVIQUAT 9030) et LUPAMIN 9010.

De préférence, les polymères cationiques sont choisis parmi ceux des familles (1), (2), (7) et (10) ci-dessus citées.

Parmi les polymères cationiques mentionnés ci-dessus, on peut utiliser de préférence les polysaccharides cationiques, notamment les celluloses et les gommes de galactomannanes cationiques, et en particulier les dérivés d'éther de cellulose quaternaires tels que les produits vendus sous la dénomination "JR 400" par la Société AMERCHOL, les cyclopolymères cationiques, en particulier les homopolymères ou copolymères de sels (par exemple chlorure) de diméthyldiallylammonium, vendus sous les dénominations MERQUAT 100, MERQUAT 550 et MERQUAT S par la société NALCO et leurs homologues de faibles poids moléculaires en poids, les polymères quaternaires de vinylpyrrolidone et de vinylimidazole, les homopolymères ou copolymères éventuellement réticulés de sels de méthacryloyloxyalkyl(C1-C4) trialkyl(C1-C4)ammonium; et leurs mélanges.

Il est également possible d'utiliser des polymères amphotères, qui peuvent de préférence être choisis parmi les polymères amphotères comprenant la répétition de :
(i) un ou plusieurs motifs issus d'un monomère de type (méth)acrylamide,
(ii) un ou plusieurs motifs issus d'un monomère de type (méth)acrylamidoalkyltrialkylammonium, et
(iii) un ou plusieurs motifs issus d'un monomère acide de type acide (méth)acrylique.

De préférence, les motifs issus d'un monomère de type (i) (méth)acrylamide sont des motifs de structure (la) suivante : 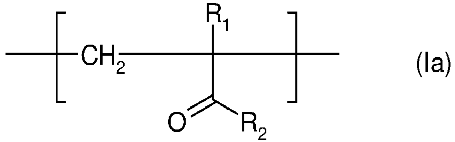 dans laquelle R₁ désigne H ou CH₃, et R₂ est choisi parmi un radical amino, diméthylamino, tert-butylamino, dodécylamino, ou -NH-CH₂OH.

De préférence, ledit polymère amphotère ne comprend la répétition que d'un seul motif de formule (la).

Le motif issu d'un monomère de type (méth)acrylamide de formule (la) dans laquelle R₁ désigne H et R₂ est un radical amino (NH2) est particulièrement préféré. Il correspond au monomère acrylamide proprement dit.

De préférence, les motifs issus d'un monomère de type (ii) (méth)acrylamidoalkyl trialkylammonium sont des motifs de structure (IIa) suivante : 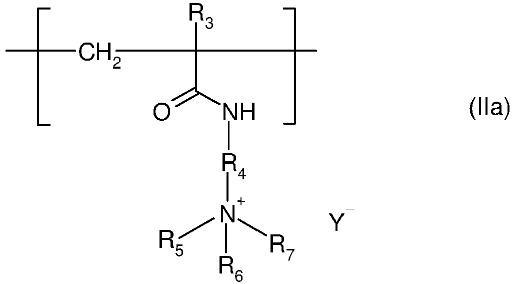 dans laquelle :
- R₃ désigne H ou CH₃,
- R₄ désigne un groupement (CH₂)k avec k un nombre entier allant de 1 à 6, et de préférence de 2 à 4 ;
- R₅, R₆ et R₇, identiques ou différents, désignent chacun un groupement alkyle ayant de 1 à 4 atomes de carbone;
- Y⁻ est un anion tel que bromure, chlorure, acétate, borate, citrate, tartrate, bisulfate, bisulfite, sulfate, phosphate.

De préférence, ledit polymère amphotère ne comprend la répétition que d'un seul motif de formule (IIa).

Parmi ces motifs issus d'un monomère de type (méth)acrylamidoalkyltrialkylammonium de formule (IIa), on préfère ceux issus du monomère chlorure de méthacrylamidopropyltriméthylammonium, pour lequel R₃ désigne un radical méthyle, k vaut 3, R₅, R₆ et R₇ désignent un radical méthyle, et Y⁻ désigne un anion chlorure.

De préférence, les motifs issus d'un monomère de type (iii) acide (méth)acrylique sont des motifs de formule (IIIa) : 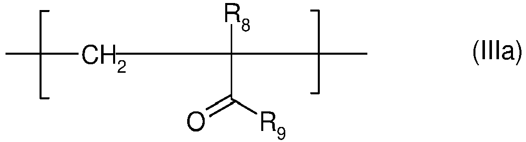 dans laquelle Rs désigne H ou CH₃, et R₉ désigne un radical hydroxyle ou un radical -NH-C(CH3₆)₂-CH₂-SO₃H.

Les motifs préférés de formules (IIIa) correspondent aux monomères acide acrylique, acide méthacrylique et acide 2-acrylamino 2-méthyl propane sulfonique.

De préférence, le motif issu d'un monomère de type acide (méth)acrylique de formule (IIIa) est celui issu de l'acide acrylique, pour lequel Rs désigne un atome d'hydrogène et R₉ désigne un radical hydroxyle.

Le ou les monomères acides de type acide (méth)acrylique peuvent être non neutralisés, ou partiellement ou totalement neutralisés par une base organique ou minérale.

De préférence, ledit polymère amphotère ne comprend la répétition que d'un seul motif de formule (IIIa).

Selon un mode de réalisation préféré de l'invention, le ou les polymères amphotères de ce type comprennent au moins 30% en mole de motifs issus d'un monomère de type (i) (méth)acrylamide. De préférence, ils comprennent de 30 à 70% en mole de motifs issus d'un monomère de type (méth)acrylamide, de manière plus préférée de 40 à 60% en mole.

La teneur en motifs issus d'un monomère de type (ii) (méth)acrylamido alkyltrialky-lammonium peut avantageusement être de 10 à 60%, préférentiellement de 20 à 55% en moles.

La teneur en motifs issus d'un monomère acide de type (iii) acide (méth)acrylique peut avantageusement être de 1 à 20%, préférentiellement de 5 à 15% en moles. Selon un mode de réalisation particulièrement préféré de l'invention, le polymère amphotère de ce type comprend :
- de 30 à 70% en moles de motifs issus d'un monomère de type (i) (méth)acrylamide, de manière plus préférée de 40 à 60% en moles,
- de 10 à 60% en moles, préférentiellement de 20 à 55% en moles, de motifs issus d'un monomère de type (ii) (méth)acrylamidoalkyltrialkylammonium, et
- de 1 à 20% en moles, préférentiellement de 5 à 15% en moles, de motifs issus d'un monomère de type (iii) acide (méth)acrylique.

Ce type de polymères amphotères peut également comprendre des motifs additionnels, différents des motifs issus d'un monomère de type (méth)acrylamide, de type (méth)acrylamidoalkyltrialkylammonium et de type acide (méth)acrylique tels que décrits ci-avant.

Toutefois, selon un mode de réalisation préféré de l'invention, lesdits polymères amphotères sont constitués uniquement de motifs issus de monomères de type (i) (méth)acrylamide, de type (ii) (méth)acrylamidoalkyltrialkylammonium et de type (iii) acide (méth)acrylique.

Comme exemple de polymères amphotères particulièrement préférés, on peut citer les terpolymères acrylamide/chlorure de méthacrylamidopropyltriméthylammonium/acide acrylique. De tels polymères sont répertoriés dans le dictionnaire C.T.F.A. International Cosmetic Ingredient Dictionary, 10ème édition 2004, sous la dénomination "Polyquaternium 53". Des produits correspondants sont notamment commercialisés sous les dénominations MERQUAT 2003 et MERQUAT 2003 PR par la société NALCO.

Comme autre type de polymère amphotère susceptible d'être utilisé, on peut également citer les copolymères à base d'acide (méth)acrylique et d'un sel de dialkyldiallylammonium, et éventuellement d'acrylamide ou d'un de ses dérivés, tels que les copolymères d'acide (méth)acrylique et de chlorure de diméthyldiallyl ammonium. On peut citer par exemple le Merquat 280 proposé par la société NALCO.

La composition selon l'invention peut comprendre les polymères cationiques et/ou amphotères en une quantité comprise entre 0,01 et 5% en poids, notamment allant de 0,05 à 3% en poids, préférentiellement de 0,1 à 2% en poids, par rapport au poids total de la composition.

### Silicone

La composition cosmétique selon l'invention peut en outre comprendre une ou plusieurs silicones, de préférence liquides, volatiles ou non volatiles.

Les silicones susceptibles d'être utilisées peuvent être solubles ou insolubles dans la composition selon l'invention; elles peuvent se présenter sous forme d'huile, de cire, de résine ou de gomme; les huiles et les gommes de silicone sont préférées.

Les silicones volatiles peuvent être choisies parmi celles possédant un point d'ébullition compris entre 60 et 260°C (à pression atmosphérique), plus particulièrement parmi :
i) les polydialkylsiloxanes cycliques comportant de 3 à 7 atomes de silicium, de préférence 4 à 5, tels que
   - l'octaméthylcyclotétrasiloxane et le décaméthylcyclopentasiloxane. On peut citer les produits commercialisés sous le nom de "VOLATILE SILICONE 7207" par UNION CARBIDE ou "SILBIONE 70045 V 2" par RHODIA, "VOLATILE SILICONE 7158" par UNION CARBIDE, "SILBIONE 70045 V 5" par RHODIA.
   - les cyclocopolymères du type diméthylsiloxane/méthylalkylsiloxane de structure chimique : 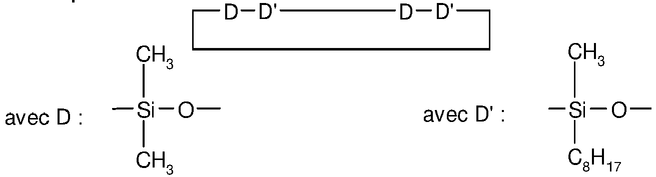 On peut citer la "SILICONE VOLATILE FZ 3109" commercialisée par la société UNION CARBIDE.
   - les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
ii) les polydialkylsiloxanes linéaires ayant 2 à 9 atomes de silicium, qui possèdent généralement une viscosité inférieure ou égale à 5.10⁻⁶ m²/s à 25°C, tels que le décaméthyltétrasiloxane.

Parmi les silicones non volatiles, on peut citer, seul ou en mélange, les polydialkylsiloxanes et notamment les polydiméthylsiloxanes (PDMS), les polydiarylsiloxanes, les polyalkylarylsiloxanes, les gommes et les résines de silicone, ainsi que les organopolysiloxanes (ou polysiloxanes organomodifiés, ou encore silicones organomodifiées) qui sont des polysiloxanes comportant dans leur structure un ou plusieurs groupements organofonctionnels, généralement fixés par l'intermédiaire d'un groupe hydrocarboné, et de préférence choisi parmi les groupements aryle, les groupements aminés, les groupements alcoxy et les groupements polyoxyéthylénés, ou polyoxypropylénés.

Les silicones organomodifiées peuvent être des polydiarylsiloxanes, notamment des polydiphénylsiloxanes, et des polyalkylarylsiloxanes fonctionnalisés par les groupes organofonctionnels mentionnés précédemment. Les polyalkylarylsiloxanes sont particulièrement choisis parmi les polydiméthyl/méthylphénylsiloxanes, les polydiméthyl /diphénylsiloxanes linéaires et/ou ramifiés.

Parmi les silicones organomodifiées, on peut citer les organopolysiloxanes comportant :
- des groupements polyoxyéthylèney et/ou polyoxypropylène comportant éventuellement des groupements alkyle en C₆-C₂₄ tels que les diméthicone-copolyols, et notamment ceux commercialisés par la société DOW CORNING sous la dénomination DC 1248 ou les huiles SILWET^{®} L 722, L 7500, L 77, L 711 de la société UNION CARBIDE; ou encore les alkyl(C₁₂)-méthicone-copolyols, et notamment ceux commercialisés par la société DOW CORNING sous la dénomination Q2-5200;
- des groupements aminés substitués ou non, en particulier des groupements aminoalkyle en C₁-C_{4;} on peut citer les produits commercialisés sous la dénomination GP4 Silicone Fluid et GP7100 par la société GENESEE, ou sous les dénominations Q2-8220 et DC929 ou DC939 par la société DOW CORNING ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX^{®} 2428, 2434 et 2440 par la société GOLDSCHMIDT ;
- des groupements hydroxylés, comme les polyorganosiloxanes à fonction hydroxyalkyle;
- des groupements acyloxyalkyle tels que les polyorganosiloxanes décrits dans le brevet US-A-4957732.
- des groupements anioniques du type acide carboxylique, comme par exemple décris dans EP186507, ou du type alkyl-carboxylique comme le produit X-22-3701E de la société SHIN-ETSU; ou encore du type 2-hydroxyalkylsulfonate ou 2-hydroxyalkylthiosulfate, comme les produits commercialisés par la société GOLDSCHMIDT sous les dénominations "ABIL^{®} S201" et "ABIL^{®} S255".
- des groupements hydroxyacylamino, comme les polyorganosiloxanes décrits dans la demande EP342834; on peut citer, par exemple, le produit Q2-8413 de la société DOW CORNING.

Les silicones peuvent également être choisies parmi les polydialkylsiloxanes parmi lesquels on peut citer principalement les polydiméthylsiloxanes à groupements terminaux triméthylsilyl. Parmi ces polydialkylsiloxanes, on peut citer les produits commerciaux suivants :
- les huiles SILBIONE^{®} des séries 47 et 70 047 ou les huiles MIRASIL^{®} commercialisées par RHODIA telles que, par exemple l'huile 70 047 V 500 000;
- les huiles de la série MIRASIL^{®} commercialisées par la société RHODIA;
- les huiles de la série 200 de la société DOW CORNING telles que la DC200 ayant viscosité 60 000 mm²/s ;
- les huiles VISCASIL^{®} de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX^{®} 9800 et 9801" par la société GOLDSCHMIDT qui sont des polydialkyl (C1-C20) siloxanes.

Des produits plus particulièrement utilisables conformément à l'invention sont des mélanges tels que :
- les mélanges formés à partir d'un polydiméthylsiloxane hydroxylé en bout de chaîne, ou dimethiconol (CTFA) et d'un polydiméthylsiloxane cyclique également appelé cyclométhicone (CTFA) tel que le produit Q2-1401 commercialisé par la société DOW CORNING.

Les polyalkylarylsiloxanes sont particulièrement choisis parmi les polydiméthyl/méthylphénylsiloxanes, les polydiméthyl /diphénylsiloxanes linéaires et/ou ramifiés de viscosité allant de 1.10⁻⁵ à 5.10-²m²/s à 25°C.

Parmi ces polyalkylarylsiloxanes, on peut citer les produits commercialisés sous les dénominations suivantes :
- les huiles SILBIONE^{®} de la série 70 641 de RHODIA;
- les huiles des séries RHODORSIL^{®} 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

La composition cosmétique selon l'invention peut comprendre lesdites silicones en une quantité allant de 0,1 à 10% en poids, mieux de 0,2 à 5% en poids, par rapport au poids total de la composition.

### Epaississants

La composition cosmétique selon l'invention peut en outre comprendre un ou plusieurs agents épaississants, de préférence non ioniques.

Au sens de la présente invention, on entend par agent épaississant un composé qui, introduit à 1% en poids dans une solution aqueuse ou hydroalcoolique à 30% d'éthanol, et à pH = 7, permet d'atteindre une viscosité d'au moins 100 cps (centipoises), de préférence d'au moins 500 cps, à 25°C et à un taux de cisaillement de 1s⁻¹. Cette viscosité peut être mesurée à l'aide d'un viscosimètre cône/plan (Rhéomètre Haake R600 ou analogue).

Les agents épaississants peuvent être choisis parmi les alcools gras éventuellement oxyalkylénés, les amides grasses, les esters d'acides gras oxyalkylénés, et les polymères épaississants acryliques, ainsi que leurs mélanges. De préférence, ils sont choisis parmi les amides grasses et les esters d'acides gras oxyalkylénés.

Les alcools gras sont de préférence de structure R-OH avec R étant un radical hydrocarboné, saturé ou insaturé, linéaire ou ramifié, comprenant 8 à 40 atomes de carbone, notamment 8 à 30 atomes de carbone, éventuellement substitué par un ou plusieurs OH, de préférence 1 à 2 OH; de préférence R est un alkyl en C8-C40, notamment en C12-C24 ; ou bien un alkenyl en C8-C40, notamment en C12-C24.

On peut notamment citer les composés suivants : lauryl alcool, cetyl alcool, stéaryle alcool, oleyl alcool, behenyl alcool, linoleyl alcool, undecylenyl alcool, palmitoleyl alcool, arachidonyl alcool et erucyl alcool, ainsi que leurs mélanges. Avantageusement, lesdits alcools gras sont solides à 25°C, c'est-à-dire ont une viscosité, mesurée avec un rhéomètre (par exemple un R600 rhéomètre), à 25°C et à un taux de cisaillement de 1 s⁻¹, supérieure ou égale à 1 Pa.s.

De préférence, les alcools gras sont choisis parmi l'alcool stéarylique, l'alcool cétylique et leurs mélanges (cetylstearyl alcool).

On entend par amide grasse, une amide comprenant dans sa structure au moins une chaine hydrocarbonée comprenant au moins 8 atomes de carbone.

Les amides grasses sont plus particulièrement choisies parmi les composés dérivant d'une amide d'alkanolamine et d'un acide gras, saturé ou insaturé, linéaire ou ramifié, comprenant 8 à 30 atomes de carbone, l'alkanolamine et/ou l'acide gras pouvant être oxyalkylénés, de préférence oxyéthylénés et comprendre 1 à 50 mol d'oxyde d'éthylène (1-50 OE).

De préférence, les amides grasses sont choisies parmi les amides d'alkanolamines en C₂-C₁₀ et d'acides gras en C₁₄-C₃₀, mieux d'alkanolamines en C₂-C₁₀ et d'acides gras en C₁₄-C₂₂.

On peut en particulier citer :
- la monoisopropanolamide de coco (ou d'acides gras de coco), telle que le produit Empilan CLS de la société Huntsman,
- la monoéthanolamide de coco (ou d'acides gras de coco), ou cocamide MEA ;
- la diethanolamide d'acides gras de soja, telle que le produit Comperlan^{®} VOD de la société Cognis,
- la monoisopropanolamide d'acide oléique, telle que le produit Witcamide^{®} 61 de la société Witco,
- la diethanolamide d'acide oléique, telle que le produit Mexanyl^{®} GT de la société Chimex,
- la monoethanolamide d'acide myristique, telle que le produit Comperlan^{®} MM de la société Cognis,
- l'ethanolamide d'acide stéarique ou la monoethanolamide d'acide stéarique, telles que les produits Monamid^{®} S et Monamid^{®} 972 de la société Uniqema,
- diethanolamide d'acide linoléique, telle que le produit Purton^{®} SFD de la société Zschimmer Schwarz,
- monoethanolamide d'acide béhénique, telle que le produit Incromide^{®} BEM de la société Croda,
- la monoisopropanolamide d'acide isostéarique, telle que le produit Witcamide^{®} SPA de la société Witco,
- la diethanolamide d'acide érucique, telle que le produit « erucic acid diethanolamide » de la société Stéarineries Dubois,
- la monoethanolamide d'acide ricinoleique, telle que le produit « ricinoleic monoethanolamide » de la société Stéarineries Dubois,
- l'amide d'acide gras du colza comprenant 4 mol OE, telle que le produit Amidet N de la société Kao.

Les esters d'acides gras oxyalkylénés peuvent être choisis parmi les dérivés oxyalkylénés d'esters d'acides gras ou d'éthers d'alcools gras.

On peut tout particulièrement citer les dérivés oxyalkylénés, notamment oxyéthylénés, d'esters d'acides gras en C₆-C₃₀ ou d'éthers d'alcools gras en C₆-C₃₀, et de polyols tels que le glycérol, le sorbitol, le glucose, le pentaerythritol ou le polyethyleneglycol, de préférence le polyéthylène glycol, ces dérivés comprenant de préférence 10 à 500 moles d'oxyde d'éthylène (OE), en particulier 30 à 400 OE, et de préférence 40 à 300 moles d'OE.

On peut en particulier citer le stéarate d'ethyleneglycol, le distéarate de polyethylene glycol à 150 OE, le stéarate de glycérol à 200 OE tel que le produit Simulsol 220 TM^{®} de SEPPIC, le tetrastearate de pentaerythritol à 150 OE, tel que le produit Crothix^{®} de Croda, le dioleate de methylglucose à 120 OE tel que le produit Glucamate DOE-120 Vegetal^{®} de la société Amerchol, le triisostearate de sorbitan à 160 OE tel que le produit Rheodol TW IS399C de la société Kao Chemicals, l'oléate de propylèneglycol à 55 OE tel que le produit Antil 141 Liquid de la société Evonik Goldschmidt.

En particulier, les esters d'acides gras oxyalkylénés peuvent répondre à la formule suivante : R₁-CO-(X)ₙ-(OCH_{2C}H₂)ₘ-O-(CO)ₚ-R₂
dans laquelle :
- R₁ est un radical alkyle ou alkényle en C₉-C₂₉ , linéaire ou ramifié,
- R₁ est un atome d'hydrogène ou un radical alkyle ou alkényle en C₉-C₂₉ , linéaire ou ramifié,
- X est un radical divalent alkylène, linéaire ou ramifié, en C₁-C₄, de préférence de formule -CH₂-CH(CH₃)- ;
- n est 0 ou 1, p est 0 ou 1 et m est un entier allant de 50 à 200.

Parmi les polymères épaississants acryliques, on préfère tout particulièrement :
- les homopolymères d'acide (méth)acrylique réticulés, tels que les produits vendus sous les noms CARBOPOLS 980, 981, 954, 2984 et 5984 par la société GOODRICH ou les produits vendus sous les noms SYNTHALEN M et SYNTHALEN K par la société 3 VSA; et
- les copolymères réticulés d'acide (méth)acrylique et d'acrylate d'alkyle en C1-C6, et notamment les copolymères réticulés d'acide méthacrylique et d'acrylate d'éthyle ou d'acide acrylique et d'acrylate d'éthyle, tels que les produits vendus sous les dénominations VISCOATEX 538C par la société COATEX, ACULYN 33 par la société ROHM & HAAS ou CARBOPOL AQUA SF-1 par la société NOVEON.

La composition cosmétique selon l'invention peut comprendre le/les agents épaississants en une quantité allant de 0,01 à 10% en poids, mieux de 0,05 à 8% en poids, encore mieux de 0,1 à 5% en poids, et préférentiellement de 0,5 à 4% en poids, par rapport au poids total de la composition.

### Autres ingrédients

La composition cosmétique selon l'invention peut se présenter sous toutes les formes galéniques classiquement utilisées, et notamment sous forme d'une solution ou suspension aqueuse, alcoolique ou hydroalcoolique, ou huileuse; d'une solution ou d'une dispersion du type lotion ou sérum; d'une émulsion, d'un gel aqueux ou anhydre, ou de toute autre forme cosmétique.

La composition selon l'invention est de préférence aqueuse et comprend alors de l'eau à une concentration allant de préférence de 30 à 98% en poids, notamment de 50 à 95% en poids, mieux de 60 à 90% en poids, par rapport au poids total de la composition.

La composition peut également comprendre un ou plusieurs solvants organiques liquides à 25°C, 1 atm., notamment hydrosolubles, tels que les alcools en C1-C6, et notamment les monoalcools aliphatiques ou aromatiques en C1-C6, les polyols en C3-C7 tels que la glycérine; les glycols tels que le butylène glycol, l'isoprène glycol, le propylène glycol; et les éthers de polyols en C3-C7, qui peuvent donc être employés seuls ou en mélange avec de l'eau. Avantageusement, le solvant organique peut être choisi parmi l'éthanol, l'isopropanol et leurs mélanges.

La composition selon l'invention peut en outre comprendre au moins un ingrédient cosmétique usuel, différent des composés de l'invention, et notamment choisi parmi les huiles végétales, minérales, animales ou de synthèse; les corps gras solides et notamment les cires, les esters en C8-C40, les acides en C8-C40; les alcools en C8-C40; les tensioactifs cationiques, les polymères anioniques; les filtres solaires; les agents hydratants; les agents antipelliculaires; les agents antioxydants; les agents chélatants; les agents nacrants et opacifiants; les agents plastifiants ou de coalescence; les hydroxyacides; les charges; les parfums; les agents d'alcalinisation ou d'acidification; les aldéhydes, la DHA; les épaississants polymériques ou non, et notamment les polymères associatifs; les conservateurs; les séquestrants (EDTA et ses sels); les matières colorantes. La composition peut bien évidemment comprendre plusieurs ingrédients cosmétiques figurant dans la liste ci-dessus. L'homme de métier veillera à choisir les ingrédients entrant dans la composition, ainsi que leurs quantités, de manière à ce qu'ils ne nuisent pas aux propriétés des compositions de la présente invention.

Le pH de la composition, si elle est aqueuse, est de préférence compris entre 4 et 7,5, notamment entre 4,5 et 6.

La composition cosmétique peut être rincée ou non rincée après avoir été appliquée sur les matières kératiniques; de préférence elle est rincée, après un éventuel temps de pose qui peut être de quelques minutes.

La composition cosmétique selon l'invention trouve notamment une application particulièrement intéressante dans le domaine de l'hygiène corporelle et/ou capillaire, notamment pour le nettoyage des cheveux et/ou du cuir chevelu, ainsi que pour le nettoyage et/ou démaquillage de la peau du corps et/ou du visage.

Elle peut ainsi constituer un shampoing ou un gel-douche, ou encore un masque à rincer.

L'invention a également pour objet un procédé de traitement cosmétique, notamment de soin et/ou de nettoyage, des matières kératiniques, notamment des cheveux, du cuir chevelu, de la peau du corps et/ou du visage, comprenant l'application sur lesdites matières kératiniques, d'une composition cosmétique selon l'invention, suivie éventuellement d'un rinçage, après un éventuel temps de pose.

L'invention concerne notamment un procédé cosmétique de nettoyage des résidus de salissure des matières kératiniques humaines, dans lequel on applique une composition selon l'invention sur lesdites matières kératiniques, en présence d'eau, on masse pour former une mousse, puis on élimine la mousse formée et les résidus de salissure par rinçage à l'eau.

L'invention est illustrée plus en détails dans les exemples suivants. Dans ces exemples, la viscosité est mesurée comme indiqué plus haut (à l'aide d'un appareil rhéomètre Haake Mars en géométrie cône-plan diamètre 60 mm /1°).

### Exemples

On prépare les compositions capillaires de type shampoing ci-après (% en poids de matière active MA) :

| | Exemple 1 | Exemple 2 | Exemple 3 |
|---|---|---|---|
| SODIUM C14-16 OLEFIN SUL-FONATE (Bioterge AS-40A de Stepan) | 12 | 5 | 15 |
| DISODIUM LAURYL SULFOSUC-CINATE | 6 | -- | -- |
| SODIUM LAUROYL SARCOSI-NATE | -- | 10 | 3 |
| COCAMIDOPROPYL BETAINE | 2 | 2 | 2 |
| COCAMIDE MEA | 0,5 | 2 | 1 |
| POL YQUATERNIUM-10 | 0,5 | 0,5 | 0,5 |
| PDMS 60 000 | 1,75 | 1,75 | 1,75 |
| CARBOMER | 0,15 | 0,15 | 0,15 |
| NaCl | 1,25 | 0,6 | 0,85 |
| Agent de pH (acide citrique, NaOH) | qsp pH 5,3 | qsp pH 5,3 | qsp pH 5,3 |
| Eau | Qsp 100% | Qsp 100% | Qsp 100% |
| Viscosité (mPa.s) | 7065 | 3360 | 6100 |
| | | | |

| | Exemple 4 | Exemple 5 | Exemple 6 |
|---|---|---|---|
| SODIUM C14-16 OLEFIN SULFONATE (Bioterge AS-40A de Stepan) | 15 | 12 | 14 |
| SODIUM LAUROYL SARCOSI-NATE | 3 | 2,4 | 2 |
| COCAMIDOPROPYL BETAINE | -- | 2 | 2 |
| COCOBETAINE | 2 | -- | -- |
| COCAMIDE MEA | 1 | 1 | 2 |
| POL YQUATERNIUM-10 | 0,5 | 0,5 | 0,5 |
| PDMS 60 000 | 1,75 | 1,75 | 1,75 |
| CARBOMER | 0,15 | 0,15 | 0,15 |
| NaCl | 0,5 | 1,25 | 0,4 |
| Agent de pH (acide citrique, NaOH) | qsp pH 5,3 | qsp pH 5,3 | qsp pH 5,3 |
| Eau | Qsp 100% | Qsp 100% | Qsp 100% |
| Viscosité (mPa.s) | 7000 | 7750 | 6300 |
| | | | |

| | Exemple 7 | Exemple 8 | Exemple 9 |
|---|---|---|---|
| SODIUM C14-16 OLEFIN SULFONATE (Bioterge AS-40A de Stepan) | 9 | 8 | 8 |
| SODIUM LAUROYL SARCOSI-NATE | 4,5 | 4 | 4 |
| COCAMIDOPROPYL BETAINE | 2 | 2 | 2 |
| COCAMIDE MEA | 1,5 | 2 | 1 |
| POL YQUATERNIUM-10 | 0,5 | 0,5 | 0,5 |
| PDMS 60 000 | 1,75 | 1,75 | 1,75 |
| CARBOMER | 0,15 | 0,15 | 0,15 |
| NaCl | 1 | 0,4 | 1,2 |
| Agent de pH (acide citrique, NaOH) | qsp pH 5,3 | qsp pH 5,3 | qsp pH 5,3 |
| Eau | Qsp 100% | Qsp 100% | Qsp 100% |
| Viscosité (mPa.s) | 6500 | 7600 | 6150 |
| | | | |

| | Exemple 10 | Exemple 11 | Exemple 12 |
|---|---|---|---|
| SODIUM C14-16 OLEFIN SUL-FONATE (Bioterge AS-40A de Stepan) | 8 | 7 | 6 |
| SODIUM LAUROYL SARCOSI-NATE | 4 | 3,5 | 3 |
| COCAMIDOPROPYL BETAINE | 2 | 2 | 2 |
| COCAMIDE MEA | 1,5 | 1,5 | 1,5 |
| POL YQUATERNIUM-10 | 0,5 | 0,5 | 0,5 |
| PDMS 60 000 | 1,75 | 1,75 | 1,75 |
| CARBOMER | 0,15 | 0,15 | 0,15 |
| NaCl | 0,75 | 0,55 | 0,5 |
| Agent de pH (acide citrique, NaOH) | qsp pH 5,3 | qsp pH 5,3 | qsp pH 5,3 |
| Eau | Qsp 100% | Qsp 100% | Qsp 100% |
| Viscosité (mPa.s) | 6700 | 6200 | 6800 |
| | | | |

| | Exemple 13 | Exemple 14 | |
|---|---|---|---|
| SODIUM C14-16 OLEFIN SULFONATE (Bioterge AS-40A de Stepan) | 10 | 7,5 | |
| SODIUM LAUROYL SARCOSINATE | 5 | 7,5 | |
| COCAMIDOPROPYL BETAINE | 2 | 2 | |
| COCAMIDE MEA | 2 | 2 | |
| POLYQUATERNIUM-10 | 0,5 | 0,5 | |
| PDMS 60 000 | 1,75 | 1,75 | |
| CARBOMER | 0,15 | 0,15 | |
| NaCl | 0,6 | 0,5 | |
| Agent de pH (acide citrique, NaOH) | qsp pH 5,3 | qsp pH 5,3 | |
| Eau | Qsp 100% | Qsp 100% | |
| Viscosité (mPa.s) | 5700 | 5270 | |

On obtient des compositions détergentes stables, présentant un bon épaississement avec une viscosité d'au moins 4 Pa.s, à effet moussant, susceptibles d'être employées pour le nettoyage des cheveux et conduisant à l'obtention de bonnes propriétés cosmétiques pour les cheveux.

Les compositions présentent un bon démarrage de mousse et génèrent une mousse abondante et de bonne qualité : la mousse présente en particulier une texture crémeuse et se répartit aisément sur l'ensemble de la chevelure.

Les exemples 4 et 7 ont été testés par un panel sensoriel de 10 consommatrices entraînées et comparés à une formule de shampoing sans sulfate du commerce.

Les deux exemples selon l'invention présentent un démarrage, une abondance, une fermeté et une tenue de mousse équivalents au produit commercial, bien que ces compositions selon l'invention comprennent beaucoup moins de tensioactif anionique (entre 10 et 30% en moins).

## Revendications

1. Composition cosmétique comprenant :
- (i) une ou plusieurs α-oléfine sulfonates linéaires,
- (ii) un ou plusieurs tensioactifs anioniques non oxyalkylénés, différents des composés (i), présents dans la composition en une quantité allant de 1 à 20% en poids par rapport au poids total de la composition; la composition comprenant au moins un tensioactif anionique non oxyalkyléné, différent des composés (i), choisi parmi les acylsarcosinates, les alkylsulfosuccinates en C6-C24, et les (C6-C24)acyliséthionates, ainsi que les sels de ces composés et leurs mélanges, et
- (iii) au moins un tensioactif non ionique choisi parmi les (alkyl C6-C24)polyglycosides, les esters d'acides gras en C8-C30 du sorbitane éthoxylés, les alcools gras en C8-C30 polyéthoxylés, les esters d'acides gras en C8-C30 polyoxyéthylénés, les alcanolamides d'acides gras en C8-C30, et leurs mélanges, et au moins un tensioactif amphotère choisi parmi les alkyl(C8-C20)bétaïnes, les alkyl(C8-C20)amidoalkyl(C1-C6)bétaïnes, les alkyl(C8-C20) amphoacétates et les alkyl(C8-C20) amphodiacétates, et leurs mélanges ; la quantité totale des tensioactifs additionnels non ioniques et amphotères allant de 1,5 à 12% en poids, par rapport au poids total de la composition ;
ladite composition ne comprenant pas de tensioactif anionique comprenant de groupement sulfate.

2. Composition selon la revendication 1, dans laquelle les α-oléfine sulfonates linéaires comprennent des alcènes sulfonates linéaires, notamment de formule (A) :
R-CH₂-CH=CH-(CH₂)n-SO₃M
dans laquelle :
- R est un radical alkyle linéaire saturé comprenant de 4 à 30 atomes de carbone, notamment de 6 à 20 atomes de carbone, voire de 8 à 18 atomes de carbone, encore mieux de 10 à 14 atomes de carbone ;
- n est un entier compris entre 0 et 10, de préférence entre 1 et 4, et encore mieux n=1 ;
- M étant un cation cosmétiquement acceptable, notamment choisi parmi le cation ammonium, les cations issus des métaux alcalins ou alcalinoterreux, les cations issus d'amines organiques comme les alcanolamines; de préférence ceux issus des métaux alcalins, et notamment Na+ ou K+.

3. Composition selon la revendication précédente, dans laquelle les alcènes sulfonates linéaires sont de formule : 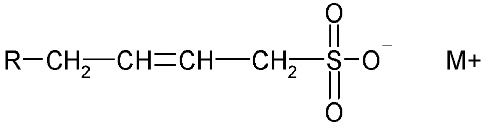 dans laquelle :
- R est un radical alkyle linéaire saturé comprenant de 4 à 20 atomes de carbone, notamment de 6 à 18 atomes de carbone, voire de 8 à 14 atomes de carbone, encore mieux de 10 à 12 atomes de carbone ;
- M étant un cation cosmétiquement acceptable, notamment choisi parmi le cation ammonium, les cations issus des métaux alcalins ou alcalinoterreux, les cations issus d'amines organiques comme les alcanolamines; de préférence ceux issus des métaux alcalins, et encore mieux Na+ ou K+.

4. Composition selon l'une des revendications précédentes, dans laquelle les α-oléfine sulfonates linéaires comprennent des hydroxyalcanes sulfonates linéaires, notamment de formule (B) :
(B) R'-CH₂-CH(OH)-CH₂-(CH₂)n'-SO₃M'
dans laquelle :
- R' est un radical alkyle linéaire saturé comprenant de 4 à 30 atomes de carbone, notamment de 6 à 20 atomes de carbone, voire de 8 à 18 atomes de carbone, en particulier 8 à 14 atomes de carbone, encore mieux de 10 à 14 atomes de carbone, notamment de 10 à 12 atomes de carbone;
- n' est un entier compris entre 0 et 10, de préférence entre 1 et 4, et encore mieux n'=1 ;
- M' étant un cation cosmétiquement acceptable, notamment choisi parmi le cation ammonium, les cations issus des métaux alcalins ou alcalinoterreux, les cations issus d'amines organiques comme les alcanolamines; de préférence ceux issus des métaux alcalins, et notamment Na+ ou K+.

5. Composition selon la revendication précédente, dans laquelle les hydroxyalcanes sulfonates linéaires sont de formule : 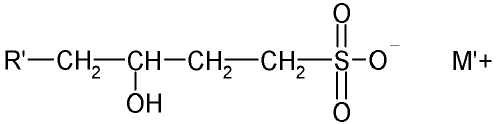 dans laquelle :
- R' est un radical alkyle linéaire saturé comprenant de 4 à 20 atomes de carbone, notamment de 6 à 18 atomes de carbone, voire de 8 à 14 atomes de carbone, encore mieux de 10 à 12 atomes de carbone ;
- M' étant un cation cosmétiquement acceptable, notamment choisi parmi le cation ammonium, les cations issus des métaux alcalins ou alcalinoterreux, les cations issus d'amines organiques comme les alcanolamines; de préférence ceux issus des métaux alcalins, et notamment Na+ ou K+.

6. Composition selon l'une des revendications précédentes, dans laquelle les α-oléfine sulfonates linéaires sont choisies parmi les α-oléfine sulfonates linéaires comprenant 8 à 28 atomes de carbone, notamment de 10 à 24 atomes de carbone, encore mieux de 12 à 20 atomes de carbone, en particulier de 14 à 18 atomes de carbone; en particulier de métaux alcalins, notamment de sodium.

7. Composition selon l'une des revendications précédentes, comprenant lesdites alpha-oléfine sulfonates linéaires en une quantité allant de 1 à 20% en poids, notamment de 4 à 18% en poids, de préférence de 6 à 16% en poids, par rapport au poids total de la composition cosmétique.

8. Composition selon l'une des revendications précédentes, comprenant lesdits tensioactifs anioniques non oxyalkylénés différents des composés (i) en une quantité allant de 1,5 à 15% en poids, mieux de 2 à 10% en poids, encore mieux de 3 à 10% en poids, par rapport au poids total de la composition.

9. Composition selon l'une des revendications précédentes, comprenant le ou lesdits tensioactifs additionnels non ioniques et/ou amphotères, en une quantité totale allant de 2 à 10% en poids, par rapport au poids total de la composition

10. Composition selon l'une des revendications précédentes, comprenant un ou plusieurs polymères choisis parmi les polymères amphotères ou cationiques, ainsi que leurs mélanges ; de préférence en une quantité comprise entre 0,01 et 5% en poids, notamment allant de 0,05 à 3% en poids, préférentiellement de 0,1 à 2% en poids, par rapport au poids total de la composition.

11. Composition selon l'une des revendications précédentes, comprenant une ou plusieurs silicones, de préférence liquides, volatiles ou non volatiles; de préférence en une quantité allant de 0,1 à 10% en poids, mieux de 0,2 à 5% en poids, par rapport au poids total de la composition.

12. Composition selon l'une des revendications précédentes, comprenant de l'eau à une concentration allant de préférence de 30 à 98% en poids, notamment de 50 à 95% en poids, mieux de 60 à 90% en poids, par rapport au poids total de la composition.

13. Composition selon l'une des revendications précédentes, comprenant un ou plusieurs agents épaississants, de préférence non ioniques ; de préférence choisis parmi les alcools gras éventuellement oxyalkylénés, les amides grasses, les esters d'acides gras oxyalkylénés, et les polymères épaississants acryliques, ainsi que leurs mélanges.

14. Procédé de traitement cosmétique, notamment de soin et/ou de nettoyage, des matières kératiniques, notamment des cheveux, du cuir chevelu, de la peau du corps et/ou du visage, comprenant l'application sur lesdites matières kératiniques, d'une composition cosmétique selon l'une quelconque des revendications précédentes, suivie éventuellement d'un rinçage, après un éventuel temps de pose.

15. Procédé cosmétique de nettoyage des résidus de salissure des matières kératiniques humaines, dans lequel on applique une composition cosmétique selon l'une quelconque des revendications 1 à 13, sur lesdites matières kératiniques, en présence d'eau, on masse pour former une mousse, puis on élimine la mousse formée et les résidus de salissure par rinçage à l'eau.

## Patentansprüche

1. Kosmetische Zusammensetzung, umfassend...
- (i) ein oder mehrere lineare α-Olefinsulfonate,
- (ii) ein oder mehrere von den Verbindungen (i) verschiedene nicht oxyalkylenierte anionische Tenside, die in der Zusammensetzung in einer Menge im Bereich von 1 bis 20 Gew.-%, bezogen auf das Gesamtgewicht der Zusammensetzung, vorliegen; wobei die Zusammensetzung mindestens ein von den Verbindungen (i) verschiedenes nicht oxyalkyleniertes anionisches Tensid umfasst, das aus Acylsarcosinaten, C6-C24-Alkylsulfosuccinaten und (C6-C24)Acylisethionaten sowie Salzen dieser Verbindungen und Mischungen davon ausgewählt ist, und
- (iii) mindestens ein nichtionisches Tensid, ausgewählt aus (C6-C24-Alkyl)polyglycosiden, ethoxylierten C8-C30-Fettsäureestern von Sorbitan, polyethoxylierten C8-C30-Fettalkoholen, polyoxyethylenierten C8-C30-Fettsäureestern, C8-C30-Fettsäurealkanolamiden und Mischungen davon, und mindestens ein amphoteres Tensid, ausgewählt aus (C8-C20)Alkylbetainen, (C8-C20)Alkylamido(C1-C6)alkylbetainen, (C8-C20)Alkylamphoacetaten und (C8-C20)Alkylamphodiacetaten und Mischungen davon; wobei die Gesamtmenge der zusätzlichen nichtionischen und amphoteren Tenside im Bereich von 1,5 bis 12 Gew.-%, bezogen auf das Gesamtgewicht der Zusammensetzung, liegt;
wobei die Zusammensetzung kein anionisches Tensid mit einer Sulfatgruppe umfasst.

2. Zusammensetzung nach Anspruch 1, wobei die linearen α-Olefinsulfonate lineare Alkensulfonate umfassen, insbesondere der Formel (A):
R-CH₂-CH=CH-(CH₂)n-SO₃M,
in der:
- R für einen gesättigten linearen Alkylrest mit 4 bis 30 Kohlenstoffatomen, insbesondere 6 bis 20 Kohlenstoffatomen oder sogar 8 bis 18 Kohlenstoffatomen und noch besser 10 bis 14 Kohlenstoffatomen steht;
- n für eine ganze Zahl zwischen 0 und 10, vorzugsweise zwischen 1 und 4, steht und noch besser n = 1;
- M für ein kosmetisch unbedenkliches Kation steht, insbesondere ausgewählt aus dem Ammoniumkation, Kationen, die sich von Alkali- oder Erdalkalimetallen ableiten, Kationen, die sich von organischen Aminen wie Alkanolaminen ableiten; vorzugsweise denjenigen, die sich von Alkalimetallen ableiten, und insbesondere Na+ und K+.

3. Zusammensetzung nach dem vorhergehenden Anspruch, wobei die linearen Alkensulfonate die folgende Formel aufweisen: 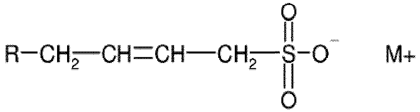 in der:
- R für einen gesättigten linearen Alkylrest mit 4 bis 20 Kohlenstoffatomen, insbesondere 6 bis 18 Kohlenstoffatomen oder sogar 8 bis 14 Kohlenstoffatomen und noch besser 10 bis 12 Kohlenstoffatomen steht;
- M für ein kosmetisch unbedenkliches Kation steht, insbesondere ausgewählt aus dem Ammoniumkation, Kationen, die sich von Alkali- oder Erdalkalimetallen ableiten, Kationen, die sich von organischen Aminen wie Alkanolaminen ableiten; vorzugsweise denjenigen, die sich von Alkalimetallen ableiten, und noch besser Na+ und K+.

4. Zusammensetzung nach einem der vorhergehenden Ansprüche, wobei die linearen α-Olefinsulfonate lineare Hydroxyalkansulfonate umfassen, insbesondere der Formel (B):
(B) R'-CH₂-CH(OH)-CH₂-(CH₂)n'-SO₃M' ,
in der:
- R'für einen gesättigten linearen Alkylrest mit 4 bis 30 Kohlenstoffatomen, insbesondere 6 bis 20 Kohlenstoffatomen oder sogar 8 bis 18 Kohlenstoffatomen, insbesondere 8 bis 14 Kohlenstoffatomen, noch besser 10 bis 14 Kohlenstoffatomen, insbesondere 10 bis 12 Kohlenstoffatomen, steht;
- n'für eine ganze Zahl zwischen 0 und 10, vorzugsweise zwischen 1 und 4, steht und noch besser n' = 1;
- M' für ein kosmetisch unbedenkliches Kation steht, insbesondere ausgewählt aus dem Ammoniumkation, Kationen, die sich von Alkali- oder Erdalkalimetallen ableiten, Kationen, die sich von organischen Aminen wie Alkanolaminen ableiten; vorzugsweise denjenigen, die sich von Alkalimetallen ableiten, und insbesondere Na+ und K+.

5. Zusammensetzung nach dem vorhergehenden Anspruch, wobei die linearen Hydroxyalkansulfonate die folgende Formel aufweisen: 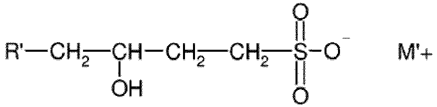 in der:
- R'für einen gesättigten linearen Alkylrest mit 4 bis 20 Kohlenstoffatomen, insbesondere 6 bis 18 Kohlenstoffatomen oder sogar 8 bis 14 Kohlenstoffatomen und noch besser 10 bis 12 Kohlenstoffatomen steht;
- M für ein kosmetisch unbedenkliches Kation steht, insbesondere ausgewählt aus dem Ammoniumkation, Kationen, die sich von Alkali- oder Erdalkalimetallen ableiten, Kationen, die sich von organischen Aminen wie Alkanolaminen ableiten; vorzugsweise denjenigen, die sich von Alkalimetallen ableiten, und insbesondere Na+ und K+.

6. Zusammensetzung nach einem der vorhergehenden Ansprüche, wobei die linearen α-Olefinsulfonate aus linearen α-Olefinsulfonaten mit 8 bis 28 Kohlenstoffatomen, insbesondere 10 bis 24 Kohlenstoffatomen, noch besser 12 bis 20 Kohlenstoffatomen, insbesondere 14 bis 18 Kohlenstoffatomen; insbesondere von Alkalimetallen, insbesondere von Natrium, ausgewählt sind.

7. Zusammensetzung nach einem der vorhergehenden Ansprüche, die die linearen alpha-Olefinsulfonate in einer Menge im Bereich von 1 bis 20 Gew.-%, insbesondere von 4 bis 18 Gew.-%, vorzugsweise von 6 bis 16 Gew.-%, bezogen auf das Gesamtgewicht der kosmetischen Zusammensetzung, umfasst.

8. Zusammensetzung nach einem der vorhergehenden Ansprüche, die die von den Verbindungen (i) verschiedenen nicht oxyalkylenierten anionischen Tenside in einer Menge im Bereich von 1,5 bis 15 Gew.-%, noch besser von 2 bis 10 Gew.-% und sogar noch besser von 3 bis 10 Gew.-%, bezogen auf das Gesamtgewicht der Zusammensetzung, umfasst.

9. Zusammensetzung nach einem der vorhergehenden Ansprüche, die das bzw. die zusätzlichen nichtionischen und/oder amphoteren Tenside in einer Gesamtmenge im Bereich von 2 bis 10 Gew.-%, bezogen auf das Gesamtgewicht der Zusammensetzung, umfasst.

10. Zusammensetzung nach einem der vorhergehenden Ansprüche, die ein oder mehrere Polymere umfasst, die aus amphoteren oder kationischen Polymeren sowie Mischungen davon ausgewählt sind; vorzugsweise in einer Menge zwischen 0,01 und 5 Gew.-%, insbesondere im Bereich von 0,05 bis 3 Gew.-%, bevorzugt von 0,1 bis 2 Gew.-%, bezogen auf das Gesamtgewicht der Zusammensetzung.

11. Zusammensetzung nach einem der vorhergehenden Ansprüche, umfassend ein oder mehrere Silikone, die vorzugsweise flüssig, flüchtig oder nichtflüchtig sind; vorzugsweise in einer Menge im Bereich von 0,1 bis 10 Gew.-%, noch besser von 0,2 bis 5 Gew.-%, bezogen auf das Gesamtgewicht der Zusammensetzung.

12. Zusammensetzung nach einem der vorhergehenden Ansprüche, die Wasser in einer Konzentration umfasst, die vorzugsweise im Bereich von 30 bis 98 Gew.-%, insbesondere von 50 bis 95 Gew.-% und noch besser von 60 bis 90 Gew.-%, bezogen auf das Gesamtgewicht der Zusammensetzung, liegt.

13. Zusammensetzung nach einem der vorhergehenden Ansprüche, umfassend ein oder mehrere Verdickungsmittel, die vorzugsweise nichtionisch sind; vorzugsweise ausgewählt aus gegebenenfalls oxyalkylenierten Fettalkoholen, Fettamiden, oxyalkylenierten Fettsäureestern, verdickenden Acrylatpolymeren sowie Mischungen davon.

14. Verfahren zur kosmetischen Behandlung, insbesondere zur Pflege und/oder Reinigung, von Keratinmaterialien, insbesondere des Haars, der Kopfhaut, der Körper- und/oder Gesichtshaut, bei dem man auf die Keratinmaterialien eine kosmetische Zusammensetzung nach einem der vorhergehenden Ansprüche aufbringt und gegebenenfalls anschließend nach einer fakultativen Einwirkungszeit spült.

15. Kosmetisches Verfahren zum Befreien menschlicher Keratinmaterialien von Schmutzresten, bei dem man eine kosmetische Zusammensetzung nach einem der Ansprüche 1 bis 13 in Gegenwart von Wasser auf die Keratinmaterialien aufbringt, zur Bildung eines Schaums massiert und dann den gebildeten Schaum und die Schmutzreste durch Spülen mit Wasser entfernt.

## Claims

1. Cosmetic composition comprising:
- (i) one or more linear α-olefin sulfonates,
- (ii) one or more non-oxyalkylenated anionic surfactants other than the compounds (i), present in the composition in an amount ranging from 1% to 20% by weight relative to the total weight of the composition; the composition comprising at least one non-oxyalkylenated anionic surfactant, other than the compounds (i), chosen from acyl sarcosinates, C6-C24 alkylsulfosuccinates and (C6-C24)acylisethionates, and also the salts of these compounds and mixtures thereof, and
- (iii) at least one nonionic surfactant chosen from (C6-C24 alkyl)polyglycosides, ethoxylated C8-C30 fatty acid esters of sorbitan, polyethoxylated C8-C30 fatty alcohols, polyoxyethylenated C8-C30 fatty acid esters, C8-C30 fatty acid alkanolamides, and mixtures thereof, and at least one amphoteric surfactant chosen from (C8-C20)alkylbetaines, (C8-C20)alkylamido(C1-C6)alkylbetaines, (C8-C20)alkyl amphoacetates and (C8-C20)alkyl amphodiacetates, and mixtures thereof; the total amount of the additional nonionic and amphoteric surfactants ranging from 1.5% to 12% by weight, relative to the total weight of the composition;
said composition not comprising any anionic surfactant comprising a sulfate group.

2. Composition according to Claim 1, in which the linear α-olefin sulfonates comprise linear alkene sulfonates, notably of formula (A):
R-CH₂-CH=CH- (CH₂)n-SO₃M
in which:
- R is a saturated linear alkyl radical comprising from 4 to 30 carbon atoms, notably from 6 to 20 carbon atoms, or even from 8 to 18 carbon atoms and better still from 10 to 14 carbon atoms;
- n is an integer between 0 and 10, preferably between 1 and 4 and better still n = 1;
- M is a cosmetically acceptable cation, chosen notably from the ammonium cation, cations derived from alkali metals or alkaline-earth metals, cations derived from organic amines such as alkanolamines; preferably those derived from alkali metals; and notably Na+ or K+.

3. Composition according to the preceding claim, in which the linear alkene sulfonates are of formula: 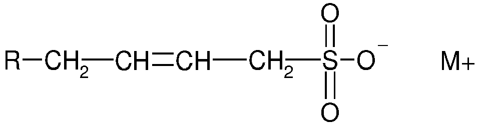 in which:
- R is a saturated linear alkyl radical comprising from 4 to 20 carbon atoms, notably from 6 to 18 carbon atoms, or even from 8 to 14 carbon atoms and better still from 10 to 12 carbon atoms;
- M is a cosmetically acceptable cation, chosen notably from the ammonium cation, cations derived from alkali metals or alkaline-earth metals, cations derived from organic amines such as alkanolamines; preferably those derived from alkali metals; and better still Na+ or K+.

4. Composition according to one of the preceding claims, in which the linear α-olefin sulfonates comprise linear hydroxyalkane sulfonates, notably of formula (B):
(B) R'-CH₂-CH(OH)-CH₂-(CH₂)n'-SO₃M'
in which:
- R'is a saturated linear alkyl radical comprising from 4 to 30 carbon atoms, notably from 6 to 20 carbon atoms, or even from 8 to 18 carbon atoms, in particular 8 to 14 carbon atoms, better still from 10 to 14 carbon atoms, notably from 10 to 12 carbon atoms;
- n'is an integer between 0 and 10, preferably between 1 and 4 and better still n' = 1;
- M' is a cosmetically acceptable cation, chosen notably from the ammonium cation, cations derived from alkali metals or alkaline-earth metals, cations derived from organic amines such as alkanolamines; preferably those derived from alkali metals; and notably Na+ or K+.

5. Composition according to the preceding claim, in which the linear hydroxyalkane sulfonates are of formula: 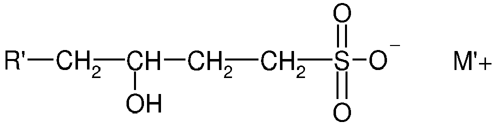 in which:
- R'is a saturated linear alkyl radical comprising from 4 to 20 carbon atoms, notably from 6 to 18 carbon atoms, or even from 8 to 14 carbon atoms and better still from 10 to 12 carbon atoms;
- M' is a cosmetically acceptable cation, chosen notably from the ammonium cation, cations derived from alkali metals or alkaline-earth metals, cations derived from organic amines such as alkanolamines; preferably those derived from alkali metals; and notably Na+ or K+.

6. Composition according to one of the preceding claims, in which the linear α-olefin sulfonates are chosen from linear α-olefin sulfonates comprising 8 to 28 carbon atoms, notably from 10 to 24 carbon atoms, better still from 12 to 20 carbon atoms, in particular from 14 to 18 carbon atoms; in particular of alkali metals, notably of sodium.

7. Composition according to one of the preceding claims, comprising said linear α-olefin sulfonates in an amount ranging from 1% to 20% by weight, notably from 4% to 18% by weight and preferably from 6% to 16% by weight, relative to the total weight of the cosmetic composition.

8. Composition according to one of the preceding claims, comprising said non-oxyalkylenated anionic surfactants other than the compounds (i) in an amount ranging from 1.5% to 15% by weight, better still from 2% to 10% by weight and even better still from 3% to 10% by weight, relative to the total weight of the composition.

9. Composition according to one of the preceding claims, comprising said additional nonionic and/or amphoteric surfactant(s) in a total amount ranging from 2% to 10% by weight, relative to the total weight of the composition.

10. Composition according to one of the preceding claims, comprising one or more polymers chosen from amphoteric or cationic polymers, and also mixtures thereof, preferably in an amount of between 0.01% and 5% by weight, notably ranging from 0.05% to 3% by weight and preferentially from 0.1% to 2% by weight, relative to the total weight of the composition.

11. Composition according to one of the preceding claims, comprising one or more silicones, which are preferably liquid, volatile or non-volatile silicones; preferably in an amount ranging from 0.1% to 10% by weight, better still from 0.2% to 5% by weight, relative to the total weight of the composition.

12. Composition according to one of the preceding claims, comprising water in a concentration preferably ranging from 30% to 98% by weight, notably from 50% to 95% by weight and better still from 60% to 90% by weight, relative to the total weight of the composition.

13. Composition according to one of the preceding claims, comprising one or more thickeners, which are preferably nonionic; preferably chosen from optionally oxyalkylenated fatty alcohols, fatty amides, oxyalkylenated fatty acid esters, and acrylic thickening polymers, and also mixtures thereof.

14. Cosmetic treatment process, notably for caring for and/or cleansing keratin materials, notably the hair, the scalp, bodily skin and/or facial skin, comprising the application to said keratin materials of a cosmetic composition according to any one of the preceding claims, optionally followed by rinsing, after an optional leaveon time.

15. Cosmetic process for cleansing soiling residues from human keratin materials, in which a cosmetic composition according to any one of Claims 1 to 13 is applied to said keratin materials in the presence of water, it is massaged to form a foam, and the foam formed and the soiling residues are then removed by rinsing with water.
